# EUROPEAN PATENT SPECIFICATION

(11) **EP 0 946 179 B1**
(45) Date of publication and mention of the grant of the patent: **17.09.2003**
(21) Application number: 96942934.9
(22) Date of filing: 04.12.1996
(51) Int. Cl.: A61K 31/5513, A61P 25/28

(54) **USE OF OLANZAPINE OR A PHARMACEUTICALLY ACCEPTABLE SALT FOR THE MANUFACTURE OF A MEDICAMENT FOR THE TREATMENT OF AUTISM AND MENTAL RETARDATION**
VERWENDUNG VON OLANZAPIN ODER EINES SALZES FÜR DIE HERSTELLUNG EINES MEDIKAMENTS FÜR DIE BEHANDLUNG VON AUTISMUS UND MENTALER RETARDATION
UTILISATION DE L'OLANZAPINE OU D'UN SEL PHARMACEUTIQUE POUR LA PREPARATION D'UN MEDICAMENT POUR LE TRAITEMENT DE L'AUTISME ET DE LA RETARDATION MENTALE.

(30) Priority: 11.03.1996 US 13162 P
(43) Date of publication of application: 06.10.1999
(73) Proprietor: ELI LILLY AND COMPANY, Indianapolis, Indiana 46285 (US)
(72) Inventor: BEASLEY, Charles, M., Jr., Indianapolis, IN 46256-2107 (US); TOLLEFSON, Gary, D., Indianapolis, IN 46236 (US)
(74) Representative: Pritchard, Judith
(86) International application number: US9619576
(87) International publication number: WO97033585

(56) References cited:
- US-A- 5 225 407
- US-A- 5 229 382
- US-A- 5 457 101
- MALEK-AHMADI P ET AL: "Olanzapine for autistic disorder with hyperactivity [letter]." JOURNAL OF THE AMERICAN ACADEMY OF CHILD AND ADOLESCENT PSYCHIATRY, (1998 SEP) 37 (9) 902., XP002108846
- BIOSIS ABSTRACTS, issued 1995, SATTERLEE et al., "A Clinical Update on Olanzapine Treatment in Schizophrenia and in Elderly Alzheimer's Disease Patients", Abstract Number 96:142562; & PSYCHOPHARMACOLOGY BULLETIN, 31(3), May/June 1995.
- CHEMICAL ABSTRACTS, issued 1995, CORBETT et al., "Antipsychotic Agents Antagonize Non-competitive N-methyl-D-aspartate Antagonist-induced Behaviors", Abstract Number 1995:752833; & PSYCHOPHARMACOLOGY BULLETIN, 31(3).

## Description

This invention provides a use of 2-methyl-4-(4-methyl-1-piperazinyl)-10H-thieno[2,3-b][1,5] benzodiazepine, (hereinafter referred as "olanzapine")for the manufacture of a medicament, for the treatment of autism and mental retardation.

Autism and mental retardation are seriously incapacitating conditions for which there has been little available treatment. The patient suffering from autism has gross and sustained impaired reciprocal social interaction. Impairment in communication is marked and sustained as well. The autistic patient suffers from restricted repetitive and stereotyped patterns of behavior, interests, and activies which are non-functional and/or abnormal in either intensity or focus. Further, the juvenile patient younger than three years old displays delays in at least one of the following areas: social interaction, language as used in social communication, or symbolic or imaginative play.

The patient suffering from mental retardation has subaverage general intellectual functioning.

Both mental retardation and autism can be debilitating conditions which may require institutionalization, if severe.

It is known that olanzapine can provide antipsychotic activity and is currently undergoing investigation for this purpose. Olanzapine is a known compound and described in U.S. Patent No. 5,229,382 as being useful for the treatment of schizophrenia, schizophreniform disorder, acute mania, mild anxiety states, and psychosis. However, olanzapine was not known to be useful for the treatment of autism and mental retardation. Applicants have discovered that olanzapine can be useful for the treatment of autism and/or mental retardation. Olanzapine could address a long felt need for treatments which provide a favorable safety profile and effectively provide relief for the patient suffering from autism and/or mental retardation.

The presently claimed invention provides a use of olanzapine or a pharmaceutically acceptable salt thereof for the manufacture of a medicament for treating autism in a mammal.

The presently claimed invention further provides a use of olanzapine or a pharmaceutically acceptable salt thereof for the manufacture of a medicament for treating mental retardation in a mammal.

Olanzapine is of the formula 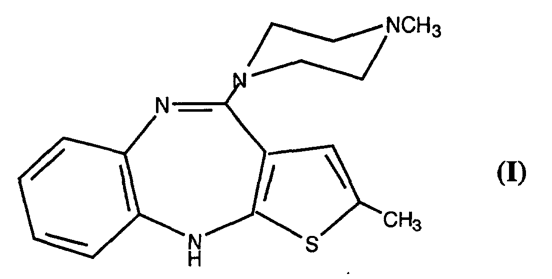 or an acid addition salt thereof.

It is especially preferred that olanzapine will be the Form II olanzapine polymorph having a typical x-ray powder diffraction pattern as represented by the following interplanar spacings:

| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

A typical example of an x-ray diffraction pattern for Form II is as follows wherein d represents the interplanar spacing and I/I₁ represents the typical relative intensities:

| **d** | **I/I**_{**1**} |
|---|---|
| 10.2689 | 100.00 |
| 8.577 | 7.96 |
| 7.4721 | 1.41 |
| 7.125 | 6.50 |
| 6.1459 | 3.12 |
| 6.071 | 5.12 |
| 5.4849 | 0.52 |
| 5.2181 | 6.86 |
| 5.1251 | 2.47 |
| 4.9874 | 7.41 |
| 4.7665 | 4.03 |
| 4.7158 | 6.80 |
| 4.4787 | 14.72 |
| 4.3307 | 1.48 |
| 4.2294 | 23.19 |
| 4.141 | 11.28 |
| 3.9873 | 9.01 |
| 3.7206 | 14.04 |
| 3.5645 | 2.27 |
| 3.5366 | 4.85 |
| 3.3828 | 3.47 |
| 3.2516 | 1.25 |
| 3.134 | 0.81 |
| 3.0848 | 0.45 |
| 3.0638 | 1.34 |
| 3.0111 | 3.51 |
| 2.8739 | 0.79 |
| 2.8102 | 1.47 |
| 2.7217 | 0.20 |
| 2.6432 | 1.26 |
| 2.6007 | 0.77 |

The x-ray diffraction patterns set out herein were obtained using a Siemens D5000 x-ray powder diffractometer having a copper K_{α} radiation source of wavelength, λ=1·541Å.

It is further preferred that the Form II olanzapine polymorph will be administered as the substantially pure Form II olanzapine polymorph.

As used herein "substantially pure" refers to Form II associated with less than about 5% Form I, preferably less than about 2% Form I, and more preferably less than about 1% Form I. Further, "substantially pure" Form II will contain less than about 0.5% related substances, wherein "related substances" refers to undesired chemical impurities or residual solvent or water. In particular, "substantially pure" Form II should contain less than about 0.05% content of acetonitrile, more preferably, less than about 0.005% content of acetonitrile. Additionally, the polymorph of the invention should contain less than 0.5% of associated water.

The polymorph obtainable by the process taught in the '382 patent will be designated as Form I and has a typical x-ray powder diffraction pattern substantially as follows, obtained using a Siemens D5000 x-ray powder diffractometer, wherein d represents the interplanar spacing:

| **d** |
|---|
| 9.9463 |
| 8.5579 |
| 8.2445 |
| 6.8862 |
| 6.3787 |
| 6.2439 |
| 5.5895 |
| 5.3055 |
| 4.9815 |
| 4.8333 |
| 4.7255 |
| 4.6286 |
| 4.533 |
| 4.4624 |
| 4.2915 |
| 4.2346 |
| 4.0855 |
| 3.8254 |
| 3.7489 |
| 3.6983 |
| 3.5817 |
| 3.5064 |
| 3.3392 |
| 3.2806 |
| 3.2138 |
| 3.1118 |
| 3.0507 |
| 2.948 |
| 2.8172 |
| 2.7589 |
| 2.6597 |
| 2.6336 |
| 2.5956 |

A typical example of an x-ray diffraction pattern for Form I is as follows wherein d represents the interplanar spacing and I/I₁ represents the typical relative intensities:

| **d** | **I/I**_{**1**} |
|---|---|
| 9.9463 | 100.00 |
| 8.5579 | 15.18 |
| 8.2445 | 1.96 |
| 6.8862 | 14.73 |
| 6.3787 | 4.25 |
| 6.2439 | 5.21 |
| 5.5895 | 1.10 |
| 5.3055 | 0.95 |
| 4.9815 | 6.14 |
| 4.8333 | 68.37 |
| 4.7255 | 21.88 |
| 4.6286 | 3.82 |
| 4.533 | 17.83 |
| 4.4624 | 5.02 |
| 4.2915 | 9.19 |
| 4.2346 | 18.88 |
| 4.0855 | 17.29 |
| 3.8254 | 6.49 |
| 3.7489 | 10.64 |
| 3.6983 | 14.65 |
| 3.5817 | 3.04 |
| 3.5064 | 9.23 |
| 3.3392 | 4.67 |
| 3.2806 | 1.96 |
| 3.2138 | 2.52 |
| 3.1118 | 4.81 |
| 3.0507 | 1.96 |
| 2.948 | 2.40 |
| 2.8172 | 2.89 |
| 2.7589 | 2.27 |
| 2.6597 | 1.86 |
| 2.6336 | 1.10 |
| 2.5956 | 1.73 |

The x-ray powder diffraction patterns herein were obtained with a copper Kₐ of wavelength 1 = 1.543Å. The interplanar spacings in the column marked "d" are in Angstroms. The typical relative intensities are in the column marked "I/I₁".

As used herein, the term "mammal" shall refer to the Mammalia class of higher vertebrates. The term "mammal" includes a human. The term "treating" as used herein includes prophylaxis of the named condition or amelioration or elimination of the condition once it has been established.

As used herein, the term "autism" shall refer to a disorder classified in the DSM-IV-R as category 299.**. Thus, including but not 299.00 (Autistic Disorder), 299.80 (Asperger's Disorder; Pervasive Development Disorder; Rett's Disorder) and 299.10 (Childhood Disintegrative Disorder). Diagnostic and Statistical Manual of Mental Disorders, Revised, 4th Ed. (1994). The use of olanzapine for the manufacture of a medicament for the treatment of disorder category 299.00 (Austistic disorder) is especially preferred. Accordingly, the term "mental retardation" shall refer to the condition characterized as such in the DSM-IV-R as catagories 317.**, 318.**, and 319. Thus, including 317 (Mild Mental Retardation), 318.0 (Moderate Mental Retardation), 318.1 (Severe Mental Retardation), 318.2 (Profound Mental Retardation) and 319(Mental Retardation, Severity Unspecified). It is especially preferred to use olanzapine for the manufacture of a medicament for the treatment of catagory 318.^{*} (Moderate Mental Retardation, Severe Mental Retardation or Profound Mental Retardation).

The DSM-IV-R was prepared by the Task Force on Nomenclature and Statistics of the American Psychiatric Association, and provides clear descriptions of diagnostic catagories. The skilled artisan will recognize that there are alternative nomenclatures, nosologies, and classification systems for pathologic psychological conditions and that these systems evolve with medical scientific progress.

The results of pharmacological studies show that olanzapine has muscarinic cholinergic receptor activity. The compound is active at the dopamine D-1 and D-2 receptors as indicated by an IC50 of less than 1 uM in the 3H-SCH233390 (Billard, et al. Life Sciences **35**:1885 (1984)) and the 3H spiperone (Seeman et al Nature **216**:717 (1976)) binding assays respectively. Further, olanzapine is active at the 5-HT-2 receptor and 5-HT1C receptor. The complex pharmacological profile of the compound provides a medicament which can be useful for the treatment of autism and/or mental retardation.

The usefulness of the compound for treating autism and/or mental retardation can be supported by the following studies as described.

### I. Clinical observations.

A double-blind multicenter clinical trial was designed to assess the safety and efficacy of olanzapine. Patients were randomized to olanzapine or placebo. The results of the study suggest that olanzapine can be useful for the treatment of autism.

Further, results of the study suggest that olanzapine can be useful for the treatment of mental retardation.

Olanzapine is effective over a wide dosage range, the actual dose to be administered being dependent on the condition being treated. For example, in adult humans, dosages of from 0.25 to 50 mg, preferably from 1 to 30 mg, and most preferably 1 to 20 mg per day may be used. A once a day dosage is normally sufficient, although divided doses may be administered. For autism, a dose range of from 1 to 30 mg, preferably 2.5 to 30 mg per day is suitable. For mental retardation, a dose range of from 1 to 30 mg per day is preferred. Radiolabelled olanzapine, can be detected in the saliva and thus the compound can potentially be monitored in patients to assess compliance.

A preferred formulation of the invention is a solid oral formulation comprising from 1 to 30 mg or 1 to 10 mg of olanzapine as an effective amount of the active ingredient.

Most preferably, the solid oral formulation is contained in packaging materials which protect the formulation from moisture and light. For example, suitable packaging materials include amber colored high density polyethylene bottles, amber colored glass bottles, and other containers made of a material which inhibits the passage of light. Most preferably, the packaging will include a desiccant pack. The container may be sealed with an aluminum foil blister to provide the desired protection and maintain product stability.

Olanzapine is normally to be administered orally or by injection and, for this purpose, it is usually employed in the form of a pharmaceutical composition.

Accordingly, pharmaceutical compositions comprising olanzapine, as active ingredient associated with a pharmaceutically acceptable carrier may be prepared. In making the compositions of the invention conventional techniques for the preparation of pharmaceutical compositions may be used. For example, the active ingredient will usually be mixed with a carrier, or diluted by a carrier, or enclosed within a carrier which may be in the form of a capsule, sachet, paper or other container. When the carrier serves as a diluent, it may be solid, semi-solid or liquid material which acts as a vehicle, excipient or medium for the active ingredient. The active ingredient can be adsorbed on a granular solid container for example in a sachet. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxy-benzoate, talc, magnesium stearate or mineral oil. The compositions of the invention may, if desired, be formulated so as to provide quick, sustained or delayed release of the active ingredient after administration to the patient.

Depending on the way of administration, the compositions for the treatment of central nervous system conditions may be formulated as tablets, capsules, injection solutions for parenteral use, gel or suspension for transdermal delivery, suspensions or elixirs for oral use or suppositories. Preferably the compositions are formulated in a unit dosage form, each dosage containing from 0.25 to 30 mg, more usually 1 to 30 mg, of the active ingredient.

The materials for the present invention can be purchased or prepared by a variety of procedures well known to those of ordinary skill in the art. Olanzapine can be prepared as described by Chakrabarti in U.S. Patent No 5,229,382 ('382). Further, the following preparations illustrate a method for preparing of the especially preferred Form II olanzapine polymorph.

Compound characterization methods include, for example, x-ray powder pattern analysis, thermogravimetric analysis (TGA), differential scanning calorimetery (DSC), titrametric analysis for water, and H¹-NMR analysis for solvent content.

The following examples are provided for purposes of illustration.

### Preparation 1

### Technical Grade olanzapine

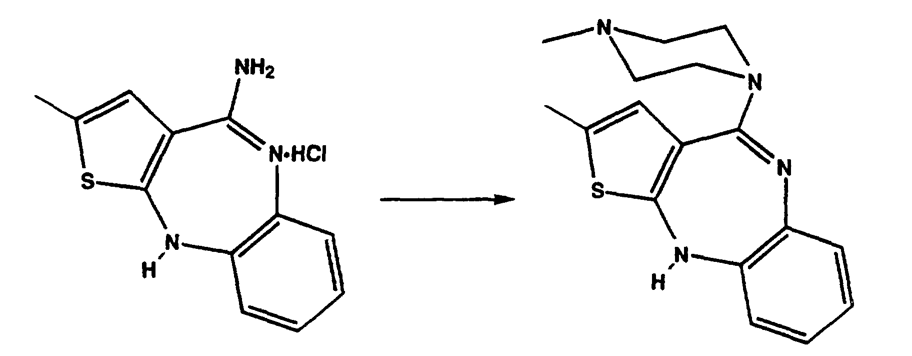

### Intermediate 1

In a suitable three neck flask the following was added:
Dimethylsulfoxide (analytical): 6 volumes
Intermediate 1 : 75 g
N-Methylpiperazine (reagent) : 6 equivalents

Intermediate 1 can be prepared using methods known to the skilled artisan. For example, the preparation of the Intermediate 1 is taught in the '382 patent.

A sub-surface nitrogen sparge line was added to remove the ammonia formed during the reaction. The reaction was heated to 120°C and maintained at that temperature throughout the duration of the reaction. The reactions were followed by HPLC until < 5% of the intermediate 1 was left unreacted. After the reaction was complete, the mixture was allowed to cool slowly to 20°C (about 2 hours). The reaction mixture was then transferred to an appropriate three neck round bottom flask and water bath. To this solution with agitation was added 10 volumes reagent grade methanol and the reaction was stirred at 20°C for 30 minutes. Three volumes of water was added slowly over about 30 minutes. The reaction slurry was cooled to zero to 5°C and stirred for 30 minutes. The product was filtered and the wet cake was washed with chilled methanol. The wet cake was dried in vacuo at 45°C overnight. The product was identified as technical olanzapine.
Yield: 76.7%; Potency: 98.1%

### Preparation 2

### Form II olanzapine polymorph

A 270 g sample of technical grade 2-methyl-4-(4-methyl-1-piperazinyl]-10H-thieno[2,3-b][1,5]benzodiazepine was suspended in anhydrous ethyl acetate (2.7 L) . The mixture was heated to 76°C and maintained at 76°C for 30 minutes. The mixture was allowed to cool to 25°C. The resulting product was isolated using vacuum filtration. The product was identified as Form II using x-ray powder analysis.
Yield: 197 g.

The process described above for preparing Form II provides a pharmaceutically elegant product having potency ≥ 97%, total related substances < 0.5% and an isolated yield of > 73%.

### EXAMPLE 1

A portion of the hydroxypropyl cellulose was dissolved in purified water to form a solution for granulation. The remaining hydroxypropyl cellulose (total of 4.0% w/w final tablet weight), which was an extra fine grade, was combined with the olanzapine (1.18% w/w), lactose (79.32% w/w) and a portion of the crospovidone (5% w/w) in a high shear granulator. All ingredients were security sieved prior to addition and dry blended in the granulator. This mixture was then granulated with the hydroxypropyl cellulose solution in the high shear granulator. The granulation was wet sized using standard methods. The wet granulation was then dried in a fluidized bed dryer and sized. The material was then added to a tumble bin mixer.
The running powders consisting of microcrystalline cellulose (granular) (10% w/w), magnesium stearate (0.5% w/w), and the remainder of the crospovidone were added to the sized granulation. The mixture was blended and compressed with the appropriate tooling on tablet compression equipment.

### Subcoating:

Hydroxypropyl methylcellulose (10% w/w) was mixed with purified water to form a solution. Core tablets were divided into approximately equal sections and spray coated with the hydroxypropyl methylcellulose solution. The operation was performed in a perforated coating pan.

### Coating of Core Tablets:

Color Mixture White (hydroxypropyl methylcellulose, polyethylene glycol, polysorbate 80, and titanium dioxide) was mixed with purified water to form the coating suspension. Subcoated tablets were divided into approximately equal sections and spray coated with the coating suspension described above. The operation was performed in a perforated coating pan.
The coated tablets were lightly dusted with carnauba wax and imprinted with appropriate identification.

## Claims

1. Use of olanzapine, or a pharmaceutically acceptable salt thereof, for the manufacture of a medicament for the treatment of autism in a mammal.

2. Use according to **Claim 1** wherein the autism is a disorder selected from Disorder, Asperger's Disorder; Pervasive Development Disorder; Rett's Disorder, and Childhood Disintegrative Disorder.

3. Use according to **Claim 2** wherein the disorder is Autistic Disorder.

4. Use according to **Claim 1** wherein olanzapine is Form II olanzapine polymorph having a typical x-ray diffraction pattern as follows, wherein d represents the interplanar spacing:
| **d(Aº)** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

5. Use according to **Claim 2** wherein olanzapine is Form II olanzapine polymorph having a typical x-ray diffraction pattern as follows, wherein d represents the interplanar spacing:
| **d(Aº)** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

6. Use according to **Claim 1** wherein the olanzapine is administrable in a dosage of from 1 mg to 30 mg per day.

7. Use according to **Claim 4** wherein the olanzapine is administrable in a dosage of from 1 mg to 30 mg per day.

8. Use of olanzapine or a pharmaceutically acceptable salt thereof, for the manufacture of a medicament for the treatment of Mental Retardation in a mammal.

9. Use according to **Claim 8** wherein the Mental Retardation is selected from Mild Mental Retardation, Moderate Mental Retardation, Severe Mental Retardation, Profound Mental Retardation, and Mental Retardation, Severity Unspecified.

10. Use according to **Claim 9** wherein the Mental Retardation is selected from Moderate Mental Retardation, Severe Mental Retardation, and Profound Mental Retardation.

11. Use according to **Claim 8** wherein olanzapine is Form II olanzapine polymorph having a typical x-ray diffraction pattern as follows, wherein d represents the interplanar spacing:
| **d(Aº)** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

12. Use according to **Claim 8** wherein the olanzapine is administrable in a dosage of from 1 mg to 30 mg per day.

13. Use according to **Claim 11** wherein the olanzapine is administrable in a dosage of from 1 mg to 30 mg per day.

## Patentansprüche

1. Verwendung von Olanzapin oder einem pharmazeutisch annehmbaren Salz hiervon zur Herstellung eines Arzneimittels für die Behandlung von Autismus bei einem Säuger.

2. Verwendung nach Anspruch 1, worin der Autismus eine Krankheit ist, welche ausgewählt ist aus autistischem Syndrom, Asperger-Syndrom, pervasivem Entwicklungssyndrom, Rett-Syndrom und disintegrativem Kindheitssyndrom

3. Verwendung nach Anspruch 2, worin die Krankheit autistisches Syndrom ist.

4. Verwendung nach Anspruch 1, worin das Olanzapin der Forln-II-Olanzapin-Polymorph mit einem typischen Röntgenbeugungsmuster wie folgt ist, worin d die Interplanarabstände bedeutet:
| **d (Å)** | **d (Å) (Fortsetzung)** |
|---|---|
| 10,2689 | 3,9873 |
| 8,577 | 3,7206 |
| 7,4721 | 3,5645 |
| 7,125 | 3,5366 |
| 6,1459 | 3,3828 |
| 6,071 | 3,2516 |
| 5,4849 | 3,134 |
| 5,2181 | 3,0848 |
| 5,1251 | 3,0638 |
| 4,9874 | 3,0111 |
| 4,7665 | 2,8739 |
| 4,7158 | 2,8102 |
| 4,4787 | 2,7217 |
| 4,3307 | 2,6432 |
| 4,2294 | 2,6007 |
| 4,141 | |

5. Verwendung nach Anspruch 2, worin das Olanzapin der Formel-II-Olanzapin-Polymorph mit einem typischen Röntgenbeugungsmuster wie folgt ist, worin d die Interplanarabstände bedeutet
| **d (Å)** | **d (Å) (Fortsetzung)** |
|---|---|
| 10,2689 | 3,9873 |
| 8,577 | 3,7206 |
| 7,4721 | 3,5645 |
| 7,125 | 3,5366 |
| 6,1459 | 3,3828 |
| 6,071 | 3,2516 |
| 5,4849 | 3,134 |
| 5,2181 | 3,0848 |
| 5,1251 | 3,0638 |
| 4,9874 | 3,0111 |
| 4,7665 | 2,8739 |
| 4,7158 | 2,8102 |
| 4,4787 | 2,7217 |
| 4,3307 | 2,6432 |
| 4,2294 | 2,6007 |
| 4,141 | |

6. Verwendung nach Anspruch 1, worin das Olanzapin in einer Dosis von 1 mg bis 30 mg pro Tag verabreichbar ist.

7. Verwendung nach Anspruch 4, worin das Olanzapin in einer Dosis von 1 mg bis 30 mg pro Tag verabreichbar ist.

8. Verwendung von Olanzapin oder einem pharmazeutisch annehmbaren Salz hiervon zur Herstellung eines Arzneimittels für die Behandlung mentaler Retardation bei einem Säuger.

9. Verwendung nach Anspruch 1, worin die mentale Retardation ausgewählt ist aus schwacher mentaler Retardation, mittlerer mentaler Retardation, starker mentaler Retardation, profunder mentaler Retardation und mentaler Retardation mit unspezifischer Stärke.

10. Verwendung nach Anspruch 9, worin die mentale Retardation ausgewählt ist aus mittlerer mentaler Retardation, starker mentaler Retardation und profunder mentaler Retardation.

11. Verwendung nach Anspruch 8, worin das Olanzapin der Form-II-Olanzapin-Polymorph mit einem typischen Röntgenbeugungsmuster wie folgt ist, worin d die Interplanarabstände bedeutet:
| **d (Å)** | **d (Å) (Fortsetzung)** |
|---|---|
| 10,2689 | 3,9873 |
| 8,577 | 3,7206 |
| 7,4721 | 3,5645 |
| 7,125 | 3,5366 |
| 6,1459 | 3,3828 |
| 6,071 | 3,2516 |
| 5,4849 | 3,134 |
| 5,2181 | 3,0848 |
| 5,1251 | 3,0638 |
| 4,9874 | 3,0111 |
| 4,7665 | 2,8739 |
| 4,7158 | 2,8102 |
| 4,4787 | 2,7217 |
| 4,3307 | 2,6432 |
| 4,2294 | 2,6007 |
| 4,141 | |

12. Verwendung nach Anspruch 8, worin das Olanzapin in einer Dosis von 1 mg bis 30 mg pro Tag verabreichbar ist.

13. Verwendung nach Anspruch 11, worin das Olanzapin in einer Dosis von 1 mg bis 30 mg pro Tag verabreichbar ist.

## Revendications

1. Utilisation d'olanzapine, ou d'un sel phannaceutiquement acceptable de celle-ci, pour la fabrication d'un médicament destiné au traitement de l'autisme chez un mammifère.

2. Utilisation selon la revendication 1, dans laquelle l'autisme est un trouble choisi parmi un trouble autistique, le trouble d'Asperger, un trouble global du développement, le trouble de Rett et un trouble désintégratif de l'enfance.

3. Utilisation selon la revendication 2, dans laquelle le trouble est un trouble autistique.

4. Utilisation selon la revendication 1, dans laquelle l'olanzapine est une olanzapine polymorphe de forme II ayant un diagramme de diffraction par rayons X typique comme suit, dans lequel d représente la distance interréticulaire :
| d(Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007 |

5. Utilisation selon la revendication 2, dans laquelle l'olanzapine est une olanzapine polymorphe de forme II ayant un diagramme de diffraction par rayons X typique comme suit, dans lequel d représente la distance interréticulaire :
| d(Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007 |

6. Utilisation selon la revendication 1, dans laquelle l'olanzapine peut être administrée selon une dose comprise dans la plage allant de 1 mg à 30 mg par jour.

7. Utilisation selon la revendication 4, dans laquelle l'olanzapine peut être administrée selon une dose comprise dans la plage allant de 1 mg à 30 mg par jour.

8. Utilisation de l'olanzapine ou d'un sel pharmaceutiquement acceptable de celle-ci, pour la fabrication d'un médicament destiné au traitement d'un retard mental chez un mammifère.

9. Utilisation selon la revendication 8, dans laquelle le retard mental est choisi parmi un retard mental léger, un retard mental modéré, un retard mental sévère, un retard mental profond et un retard mental de sévérité non spécifiée.

10. Utilisation selon la revendication 9, dans laquelle le retard mental est choisi parmi un retard mental modéré, un retard mental sévère et un retard mental profond.

11. Utilisation selon la revendication 8, dans laquelle l'olanzapine est une olanzapine polymorphe de forme II ayant un diagramme de diffraction par rayons X typique comme suit, dans lequel d représente la distance interréticulaire :
| d(Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007 |

12. Utilisation selon la revendication 8, dans laquelle l'olanzapine peut être administrée selon une dose comprise dans la plage allant de 1 mg à 30 mg par jour.

13. Utilisation selon la revendication 11, dans laquelle l'olanzapine peut être administrée selon une dose comprise dans la plage allant de 1 mg à 30 mg par jour.
